# EUROPEAN PATENT APPLICATION

(11) **EP 2 765 200 A1**
(43) Date of publication of application: **13.08.2014**
(21) Application number: 13154355.5
(22) Date of filing: 07.02.2013
(51) Int. Cl.: C12P 19/28, C07H 19/06

(54) **Process for producing gougerotin employing Streptomyces microflavus strains**

(71) Applicant: Bayer CropScience LP, Davis, California 95618 (US)
(72) Inventor: The designation of the inventor has not yet been filed
(74) Representative: Schiweck, Weinzierl & Koch

(57) **Abstract**

The invention relates to a fermentation broth obtained by cultivating a gougerotin producing *Streptomyces* strain, wherein the fermentation broth contains at least about 1 g/L gougerotin. The invention also relates to a method of producing a fermentation broth of a gougerotin producing Streptomyces strain, wherein the fermentation broth contains at least about 1 g/L gougerotin, the method comprising cultivating the Streptomyces strain in a culture medium containing a digestible carbon source and a digestible nitrogen source under aerobic conditions, wherein the culture medium contains an amino acid at a concentration effective to achieve a gougerotin concentration of at least 1 g/L.

## Description

### FIELD OF INVENTION

The present invention relates to the field of bacterial strains and their ability to control plant diseases and pests.

### BACKGROUND OF INVENTION

Phytophagous mites, especially spider mites, are a major agricultural pest of orchards, greenhouses and many vegetable and fruit crops, including peppers, tomatoes, potatoes, squash, eggplant, cucumber and strawberries. Mites damage leaf and/or fruit surfaces using their sharp mouthparts. Besides direct damage to plant parts (referred to as stippling), mite feeding also causes increased susceptibility to plant diseases.

Mites are acari rather than insects, and few broad spectrum insecticides are also effective against mites. Characteristics of mites and of available miticides pose challenges to mite control. For example, spider mites, one of the most economically important families of mites, generally live on the undersides of leaves of plants, such that they are difficult to treat. Further, mites are known to develop resistance to presently available miticides, many of which have a single mode of action, within two to four years. Few available miticides have activity against mite eggs, making repeat applications necessary. Therefore, there is a need for new miticides having translaminar, ovicidal and strong residual activities in addition to good knockdown activity.

### SUMMARY OF INVENTION

The present invention provides the *Streptomyces microflavus* strain NRRL B-50550 or a phytophagous-miticidal mutant (strain) derived therefrom.

The present invention also provides a composition containing *Streptomyces microflavus* strain NRRL B-50550 or a phytophagous-miticidal mutant (strain) derived therefrom. In one aspect, the composition is a fermentation product of the *Streptomyces microflavus* strain NRRL B-50550 or a phytophagous-miticidal mutant strain derived therefrom.

The present invention also provides a fermentation broth obtained by cultivating a gougerotin producing *Streptomyces* strain, wherein the fermentation broth contains at least about 1 g/L gougerotin.

The present invention also provides a method of producing a fermentation broth of a gougerotin producing *Streptomyces* strain, wherein the fermentation broth contains at least about 1 g/L gougerotin, the method comprising cultivating the Streptomyces strain in a culture medium containing a digestible carbon source and a digestible nitrogen source under aerobic conditions, wherein the culture medium contains an amino acid at a concentration effective to achieve a gougerotin concentration of at least 1 g/L.The present invention also provides a method of treating a plant to control a plant disease or pest, wherein the method comprises applying the *Streptomyces microflavus* strain NRRL-50550 or a phytophagous-miticidal mutant strain derived therefrom, to the plant, to a part of the plant and/or to a locus of the plant. In one embodiment, a fermentation product of the strain or a fermentation product of a mutant derived therefrom is applied to the plant and/or to a locus of the plant.

The invention also provides for a method of controlling phytophagous acari or insects comprising applying to a plant or to soil surrounding the plant the *Streptomyces microflavus* strain NRRL B-50550 or a phytophagous-miticidal strain derived therefrom. In one embodiment, a fermentation product of the strain or a fermentation product of a mutant derived thereform is applied to the plant and/or to a locus of the plant.

### BRIEF DESCRIPTION OF THE DRAWINGS

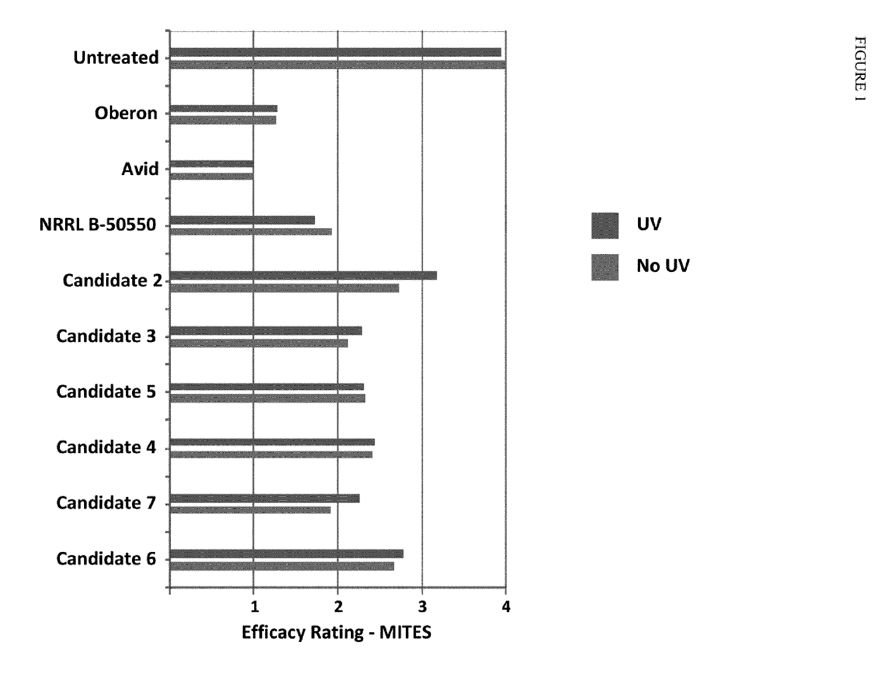

**Figure 1** shows the results of UV stability tests with bacterial candidate strains, when diluted fermentation products of the strains were sprayed on leafs of lima bean plants infested with two spotted spider mites. The columns in light gray (lower column for each strain) represent the antimiticidal activity without UV light irradiation, the columns in dark gray (upper column for each strain) represent the antimiticidal activity after irradiation with UV light for 24 hours. On the fifth day, plants were assessed for presence of mites and eggs on a scale of 1 to 4.

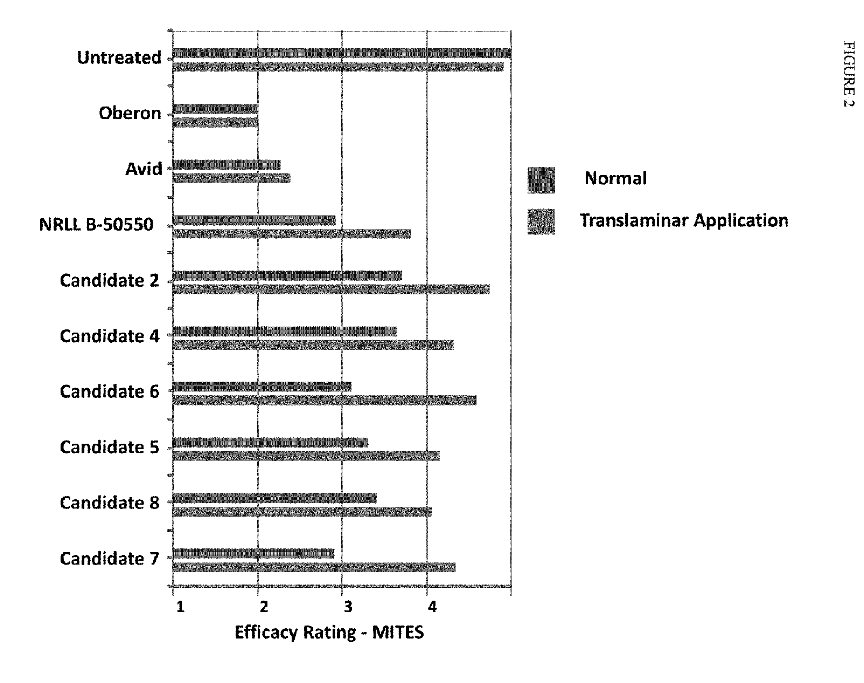

**Figure 2** shows the result of tests for translaminar activity of the bacterial candidate strains tested, when diluted fermentation products of the strains were sprayed on leafs of lima bean plants infested with two spotted spider mites. The columns in light gray (lower column for each strain) represent the translaminar (antimiticidal) activity, the columns in dark gray (upper column for each strain) represent the overall antimiticidal activity. On the sixth day, plants were assessed for presence of mites and eggs on a scale of 1 to 4.

### DETAILED DESCRIPTION OF INVENTION

All publications, patents and patent applications, including any drawings and appendices, herein are incorporated by reference to the same extent as if each individual publication or patent application was specifically and individually indicated to be incorporated by reference.

The following description includes information that may be useful in understanding the present invention. It is not an admission that any of the information provided herein is prior art or relevant to the presently claimed inventions, or that any publication specifically or implicitly referenced is prior art.

The present invention provides the *Streptomyces microflavus* strain NRRL B-50550 or a phytophagous-miticidal mutant strain derived therefrom. It has been found that the strain NRRL B-50550 has a variety of advantageous properties. Not only does the strain NRRL B-50550 (or its fermentation product) have acaricidal activity as such but, for example, also shows a high UV stability, a good translaminar activity, good ovicidal activity, long residual activity, drench activity as well as activity against a broad range of mites (see Example Section) and thus meets the requirements for an effective acaricide. In addition, the strain NRRL B-50550 (or its fermentation product) possesses both insecticidal activity and activity against various fungal phytopathogens such as leaf rust and mildew. This unique combination of activities makes the strain NRRL B-50550 a highly versatile candidate and renders the strain suitable to be broadly employed in methods of treating plants to control a plant disease and/or a plant pest. Such a broad range of activities and possible applications in agriculture has not yet been reported for known *Streptomyces* strains. In relation to a possible agricultural use, streptomyces strains have been predominantly described in publications from the late 1960's and early 1970's. *See,* for example, the British Patent No. GB 1 507 193 that describes the *Streptomyces rimofaciens* strain No. B-98891, deposited as ATCC 31120, which produces the antibiotic B-98891. According to GB 1 507 193, filed March 1975, the antibiotic B-98891 is the active ingredient that provides antifungal activity of the *Streptomyces rimofaciens* strain No. B-98891 against powdery mildew. U.S. Patent No. 3,849,398, filed August 2, 1972, describes that the strain *Streptomyces toyocaensis* var. aspiculamyceticus produces the antibiotic aspiculamycin which is also known as gougerotin (*see,* Toru Ikeuchi et al., 25 J. ANTIBIOTICS 548 (Sept. 1972). According to U.S. Patent No. 3,849,398, gougerotin has parasiticidal action against parasites on animals, such as pin worm and the like, although gougerotin is said to show a weak antibacterial activity against gram-positive, gram-negative bacteria and tubercule bacillus. Similarly, Japanese Patent Application No. JP 53109998 (A), published 1978, reports the strain *Streptomyces toyocaensis* (LA-681) and its ability to produce gougerotin for use as miticide. However, it is to be noted that no miticidal product based on such *Streptomcyes* strains is commercially available. Thus, the *Streptomyces microflavus* strain NRRL B-50550 with its broad efficacy against acari (based on gougerotin production), fungi and insects and its favorable properties in terms of mode of action (e.g., translaminar activity and residual activity) represents a significant and unexpected advancement in terms of biological and advantageous properties which as such have not been reported for known *Streptomyces* strains. Additionally, Applicant has solved the problem of producing a fermentation broth containing high concentrations of gougerotin, making feasible the ultimate use of the fermentation broth as a commercial pesticide or as a source of gougerotin for use as a commercial pesticide. Thus, this invention encompasses fermentation broths containing gougerotin at concentrations of at least about 1 g/L, at least about 2 g/L, at least about 3 g/L, at least about 4 g/L, at least about 5 g/L at least about 6 g/L, at least about 7 g/L or at least about 8 g/L. In other embodiments the fermentation broth contains gougerotin in a concentration ranging from about 2 g/L to about 15 g/L, including in a concentration of about 3g/L, of about 4 g/l, of about of about 5g/L, of about 6 g/L, of about 7 g/L, of about 8 g/L, of about 9 g/L, of about of 10 g/L, of about 11 g/L, of about 12 g/L, of about 13 g/l, and of about 14 g/L. In some embodiments the fermentation broths are from *Streptomyces* species. In specific embodiments, the fermentation broths are from *Streptomyces* microflavus. In still other specific embodiments, the fermentation broths are from *Streptomyces* microflavus NRRL-50550 or phytophagous-miticidal mutants derived therefrom. See structure of gougerotin below.

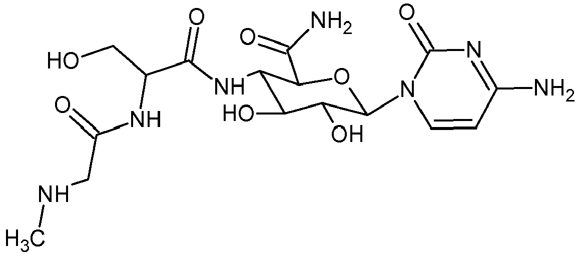

The microorganisms and particular strains described herein, unless specifically noted otherwise, are all separated from nature (i.e., isolated) and grown under artificial conditions, such as in shake flask cultures or through scaled-up manufacturing processes, such as in bioreactors, as described herein.

In one embodiment, a phytophagous-miticidal mutant strain of the *Streptomyces microflavus* strain NRRL B-50550 is provided. The term "mutant" refers to a genetic variant derived from *Streptomyces microflavus* strain NRRL B-50550. In one embodiment, the mutant has one or more or all the identifying (functional) characteristics of *Streptomyces microflavus* strain NRRL B-50550. In a particular instance, the mutant or a fermentation product thereof controls (as an identifying functional characteristic) mites at least as well as the parent *Streptomyces microflavus* NRRL B-50550 strain. In addition, the mutant or a fermentation product thereof may have one, two, three, four or all five of the following characteristics: translaminar activity in relation to the miticidal activity, residual activity in relation to the miticidal activity, ovicidal activity, insecticide activity, in particular against diabrotica, or activity against fungal phytopathogens, in particular against mildew and rust disease. Such mutants may be genetic variants having a genomic sequence that has greater than about 85%, greater than about 90%, greater than about 95%, greater than about 98%, or greater than about 99% sequence identity to *Streptomyces microflavus* strain NRRL B-50550. Mutants may be obtained by treating *Streptomyces microflavus* strain NRRL B-50550 cells with chemicals or irradiation or by selecting spontaneous mutants from a population of NRRL B-50550 cells (such as phage resistant or antibiotic resistant mutants) or by other means well known to those practiced in the art.

Suitable chemicals for mutagenesis of *Streptomcyes microflavus* include hydroxylamine hydrochloride, methyl methanesulfonate (MMS), ethyl methanesulfonate (EMS), 4-nitroquinoline 1-oxide (NQO), mitomycin C or N-methyl-N'-nitro-N-nitrosoguanidine (NTG), to mention only a few (cf., for example, Stonesifer & Baltz, Proc. Natl. Acad. Sci. USA Vol. 82, pp. 1180-1183, February 1985). The mutagenesis of *Streptomyces* strains by, for example, NTG, using spore solutions of the respective *Streptomcyes* strain is well known to the person skilled in the art. *See,* for example Delic et al, Mutation Research/Fundamental and Molecular Mechanisms of Mutagenesis, Volume 9, Issue 2, February 1970, pages 167-182, or Chen et al., J Antibiot (Tokyo), 2001 Nov; 54(11), pages 967-972.). In more detail, *Streptomyces microflavus* can be subjected to mutation by NTG using the protocol described in Kieser, T., et al., 2000, supra. Practical Streptomyces Genetics, Ch. 5 John Innes Centre, Norwich Research Park, England (2000), pp. 99-107. Mutagenesis of spores of *Streptomyces microflavus* by ultraviolet light (UV) can be carried out using standard protocols. For example, a spore suspension of the *Streptomyces* strain (freshly prepared or frozen in 20% glycerol) can be suspended in a medium that does not absorb UV light at a wave length of 254 nm (for example, water or 20% glycerol are suitable). The spore suspension is then placed in a glass Petri dish and irradiated with a low pressure mercury vapour lamp that emits most of its energy at 254 nm with constant agitation for an appropriate time at 30 °C (the most appropriate time of irradiation can be determined by first plotting a dose-survival curve). Slants or plates of non-selective medium can, for example, then be inoculated with the dense irradiated spore suspension and the so obtained mutant strains can be assessed for their properties as explained in the following. *See* Kieser, T., et al. 2000, supra.

The mutant strain can be any mutant strain that has one or more or all the identifying characteristics of *Streptomyces microflavus* strain NRRL B-50550 and in particular miticidal activity that is comparable or better than that of *Streptomyces microflavus* NRRL B-50550. The miticidal activity can, for example, be determined against two-spotted spider mites ("TSSM") as explained in Example 2 herein, meaning culture stocks of the mutant strain of *Streptomyces microflavus* NRRL B-50550 can be grown in 1 L shake flasks in Media 1 or Media 2 of Example 2 at 20-30 °C for 3-5 days, and the diluted fermentation product can then be applied on top and bottom of lima bean leaves of two plants, after which treatment, plants can be infested on the same day with 50-100 TSSM and left in the greenhouse for five days.

In one aspect, of the invention, the *Streptomyces microflavus* strain NRRL B-50550 or a phytophagous-miticidal mutant strain thereof has translaminar activity. The term "translaminar activity" is used herein in its regular meaning in the art and thus by "translaminar activity" is meant the ability of a compound or composition (here a composition such as a fermentation product containing the *Streptomyces microflavus* strain NRRL B-50550 or a mutant strain thereof) of moving through the leaf tissue of the plant to be treated. A translaminar compound/composition penetrates leaf tissues and forms a reservoir of active ingredient within the leaf. This translaminar activity therefore also provides residual activity against foliar-feeding insects and mites. Because the composition (or its one or more active ingredients) can move through leaves, thorough spray coverage is less critical to control acari such as mites, which normally feed on leaf undersides. The translaminar activity of a mutant strain alone or in comparison to *Streptomyces microflavus* NRRL B-50550 can, for example, be determined against two-spotted spider mites ("TSSM") as explained in Example 6 herein.

In another aspect of the invention, the *Streptomyces microflavus* strain NRRL B-50550 or a phytophagous-miticidal mutant strain thereof has residual activity. The term "residual activity" is used herein in its regular meaning in the art and thus by "residual activity" is meant the ability of a compound or composition (here a composition such as a fermentation product containing the *Streptomyces microflavus* strain NRRL B-50550 or a mutant strain thereof) to remain effective for an extended period of time after it is applied. The length of time may depend on the formulation (dust, liquid, etc.), the type of plant or location and the condition of the plant surface or soil surface (wet, dry, etc.) to which a composition containing *Streptomyces microflavus* strain NRRL B-50550 or a mutant strain thereof is applied. The residual activity of a mutant strain alone or in comparison to *Streptomyces microflavus* NRRL B-50550 can, for example, be determined against two-spotted spider mites ("TSSM") as explained in Example 2 or 7 herein and means, in relation to the miticidal effect, that an antimiticidal effect can still be observed after several days (e.g., 12 days) under the conditions of Example 2 or 5.

In another aspect of the invention, the *Streptomyces microflavus* strain NRRL B-50550 or a phytophagous-miticidal mutant strain thereof has ovicidal activity. The term "ovicidal activity" is used herein in its regular meaning in the art to mean "the ability of causing destruction or death of an ovum" and is used herein in relation to eggs of acari such as mites. The ovicidal activity of a mutant strain of *Streptomyces microflavus* NRRL B-50550 alone or in comparison to *Streptomyces microflavus* NRRL B-50550 can be determined using the method as described in Example 7.

In another aspect of the invention, the *Streptomyces microflavus* strain NRRL B-50550 or a phytophagous-miticidal mutant strain thereof may have drench activity. The term "drench activity" is used herein in its regular meaning in the art to mean pesticidal activity that travels from soil or other growth media upward through the plant via the xylem. The drench activity of a mutant strain of *Streptomyces microflavus* NRRL B-50550 alone or in comparison to *Streptomyces microflavus* NRRL B-5055 can be determined using the method as described in Example 8.

In another aspect of the invention, the *Streptomyces microflavus* strain NRRL B-50550 or a phytophagous-miticidal mutant strain thereof has miticidal activity against a variety of mite species, including, as illustrated in the Examples, but not limited to, activity against two-spotted spider mites, activity against citrus rust mites (*Phyllocoptruta oleivora*), eriophyid (russet) mites and broad mites.

The *Streptomyces microflavus* strain NRRL B-50550 or a phytophagous-miticidal mutant strain thereof may thus have activity against a mite that is selected from the group consisting of clover mite, brown mite, hazelnut spider mite, asparagus spider mite, brown wheat mite, legume mite, oxalis mite, boxwood mite, Texas citrus mite, Oriental red mite, citrus red mite, European red mite, yellow spider mite, fig spider mite, Lewis spider mite, six-spotted spider mite, Willamette mite, Yuma spider mite, web-spinning mite, pineapple mite, citrus green mite, honey-locust spider mite, tea red spider mite, southern red mite, avocado brown mite, spruce spider mite, avocado red mite, Banks grass mite, carmine spider mite, desert spider mite, vegetable spider mite, tumid spider mite, strawberry spider mite, two-spotted spider mite, McDaniel mite, Pacific spider mite, hawthorn spider mite, four-spotted spider mite, Schoenei spider mite, Chilean false spider mite, citrus flat mite, privet mite, flat scarlet mite, white-tailed mite, pineapple tarsonemid mite, West Indian sugar cane mite, bulb scale mite, cyclamen mite, broad mite, winter grain mite, red-legged earth mite, filbert big-bud mite, grape erineum mite, pear blister leaf mite, apple leaf edgeroller mite, peach mosaic vector mite, alder bead gall mite, Perian walnut leaf gall mite, pecan leaf edgeroll mite, fig bud mite, olive bud mite, citrus bud mite, litchi erineum mite, wheat curl mite, coconut flower and nut mite, sugar cane blister mite, buffalo grass mite, bermuda grass mite, carrot bud mite, sweet potato leaf gall mite, pomegranate leaf curl mite, ash sprangle gall mite, maple bladder gall mite, alder erineum mite, redberry mite, cotton blister mite, blueberry bud mite, pink tea rust mite, ribbed tea mite, grey citrus mite, sweet potato rust mite, horse chestnut rust mite, citrus rust mite, apple rust mite, grape rust mite, pear rust mite, flat needle sheath pine mite, wild rose bud and fruit mite, dryberry mite, mango rust mite, azalea rust mite, plum rust mite, peach silver mite, apple rust mite, tomato russet mite, pink citrus rust mite, cereal rust mite, rice rust mite and combinations thereof.

In another aspect of the invention, the *Streptomyces microflavus* strain NRRL B-50550 or a phytophagous-miticidal mutant strain thereof may have also insecticide activity. The target insect may be a diabrotica. The diabrotica may be Banded cucumber beetle (*Diabrotica balteata*), Western spotted cucumber beetle (*Diabrotica undecimpunctata undecimpunctata*), or a corn rootworm such as Northern corn rootworm (*Diabrotica barberi*), Southern corn rootworm (*Diabrotica undecimpunctata howardi*), Western cucumber beetle (*Diabrotica undecimpunctata tenella*), Western corn rootworm (*Diabrotica virgifera virgifera*), Mexican corn rootworm (*Diabrotica virgifera zeae*) and combinations of such diabrotica. The insecticidal activity of a mutant strain of *Streptomyces microflavus* NRRL B-50550 alone or in comparison to *Streptomyces microflavus* NRRL B-50550 can be determined against corn rootworm, using the method as described in Example 10.

In another aspect of the invention, the *Streptomyces microflavus* strain NRRL B-50550 or a phytophagous-miticidal mutant strain thereof has fungicide activity, meaning activity against a plant disease that is caused by a fungus. The plant disease may be mildew or a rust disease. Examples of mildew that can be treated with the *Streptomyces microflavus* strain NRRL B-50550 or a phytophagous-miticidal mutant strain thereof include, but are not limited to, powdery mildew, such as cucumber powdery mildew caused by *Sphaerotheca fuliginea,* or downy mildew, such as brassica downy mildew, caused by *Peronospora parasitica.* Examples of a rust disease that may be treated with *Streptomyces microflavus* strain NRRL B-50550 or a phytophagous-miticidal mutant strain thereof include, but are not limited to, wheat leaf rust caused by Puccinia triticina (also known as P. recondita), wheat stem rust caused by Puccinia grammis, wheat stripe rust caused by Puccinia striiformis, leaf rust of barley caused by Puccinia hordei, leaf rust of rye caused by Puccinia recondita, brown leaf rust, crown rust, and stem rust. The fungicidal activity of a mutant strain of *Streptomyces microflavus* NRRL B-50550 alone or in comparison to *Streptomyces microflavus* NRRL B-50550 can be determined against cucumber powdery mildew using the method as described in Example 9.

The present invention also encompasses methods of treating a plant to control plant pests and diseases by administering to a plant or a plant part, such as a leaf, stem, flowers, fruit, root, or seed or by applying to a locus on which plant or plant parts grow, such as soil, one or more of a gougerotin containing fermentation broth of *Streptomcyes,* the *Streptomyces microflavus* strain NRRL B-50550 or a phytophagous-miticidal mutant strain thereof or cell-free preparations thereof or metabolites thereof.

As used herein, the term "plant" refers to any living organism belonging to the kingdom Plantae (i.e., any genus/species in the Plant Kingdom). This includes familiar organisms such as but not limited to trees, herbs, bushes, grasses, vines, ferns, mosses and green algae. The term refers to both monocotyledonous plants, also called monocots, and dicotyledonous plants, also called dicots. The plant is in some embodiments of economic importance. In some embodiments the plant is a men-grown plant, for instance a cultivated plant, which may be an agricultural, a silvicultural or a horticultural plant. Examples of particular plants include but are not limited to corn, potatoes, roses, apple trees, sunflowers, wheat, rice, bananas, tomatoes, opo, pumpkins, squash, beans (e.g., lima beans), lettuce, cabbage, oak trees, guzmania, geraniums, hibiscus, clematis, poinsettias, sugarcane, taro, duck weed, pine trees, Kentucky blue grass, zoysia, coconut trees, brassica leafy vegetables (e.g., broccoli, broccoli raab, Brussels sprouts, cabbage, Chinese cabbage (Bok Choy and Napa), cauliflower, cavalo, collards, kale, kohlrabi, mustard greens, rape greens, and other brassica leafy vegetable crops), bulb vegetables (e.g., garlic, leek, onion (dry bulb, green, and Welch), shallot, and other bulb vegetable crops), citrus fruits (e.g., grapefruit, lemon, lime, orange, tangerine, citrus hybrids, pummelo, and other citrus fruit crops), cucurbit vegetables (e.g., cucumber, citron melon, edible gourds, gherkin, muskmelons (including hybrids and/or cultivars of cucumis melons), watermelon, cantaloupe, and other cucurbit vegetable crops), fruiting vegetables (including eggplant, ground cherry, pepino, pepper, tomato, tomatillo, and other fruiting vegetable crops), grape, leafy vegetables (e.g., romaine), root/tuber and corm vegetables (e.g., potato), lentils, alfalfa sprouts, clover and tree nuts (almond, pecan, pistachio, and walnut), berries (e.g., tomatoes, barberries, currants, elderberries, gooseberries, honeysuckles, mayapples, nannyberries, Oregon-grapes, see-buckthorns, hackberries, bearberries, lingonberries, strawberries, sea grapes, blackberries, cloudberries, loganberries, raspberries, salmonberries, thimbleberries, and wineberries), cereal crops (e.g., corn, rice, wheat, barley, sorghum, millets, oats, ryes, triticales, buckwheats, fonio, and quinoa), pome fruit (e.g., apples, pears), stone fruits (e.g., coffees, jujubes, mangos, olives, coconuts, oil palms, pistachios, almonds, apricots, cherries, damsons, nectarines, peaches and plums), vine (e.g., table grapes, wine grapes), fibber crops (e.g., hemp, cotton), ornamentals, to name a few. The plant may, in some embodiments, be a household/domestic plant, a greenhouse plant, an agricultural plant, or a horticultural plant. As already indicated above, in some embodiments the plant may a hardwood such as one of acacia, eucalyptus, hornbeam, beech, mahogany, walnut, oak, ash, willow, hickory, birch, chestnut, poplar, alder, maple, sycamore, ginkgo, a palm tree and sweet gum. In some embodiments the plant may be a conifer such as a cypress, a Douglas fir, a fir, a sequoia, a hemlock, a cedar, a juniper, a larch, a pine, a redwood, spruce and yew. In some embodiments the plant may be a fruit bearing woody plant such as apple, plum, pear, banana, orange, kiwi, lemon, cherry, grapevine, papaya, peanut, and fig. In some embodiments the plant may be a woody plant such as cotton, bamboo and a rubber plant. The plant may in some embodiments be an agricultural, a silvicultural and/or an ornamental plant, i.e. a plant which is commonly used in gardening, e.g., in parks, gardens and on balconies. Examples are turf, geranium, pelargonia, petunia, begonia, and fuchsia, to name just a few among the vast number of ornamentals. The term "plant" is also intended to include any plant propagules.

The term "plant" generally includes a plant that has been modified by one or more of breeding, mutagenesis and genetic engineering. Genetic engineering refers to the use of recombinant DNA techniques. Recombinant DNA techniques allow modifications which cannot readily be obtained by cross breeding under natural circumstances, mutations or natural recombination. In some embodiments a plant obtained by genetic engineering may be a transgenic plant.

As used herein, the term "plant part" refers to any part of a plant including but not limited to the shoot, root, stem, seeds, stipules, leaves, petals, flowers, ovules, bracts, branches, petioles, internodes, bark, wood, tubers, pubescence, tillers, rhizomes, fronds, blades, pollen, stamen, microspores, fruit and seed. The two main parts of plants grown in typical media employed in the art, such as soil, are often referred to as the "above-ground" part, also often referred to as the "shoots", and the "below-ground" part, also often referred to as the "roots".

In a method according to the invention a composition containing *Streptomyces microflavus* NRRL B-50550 or a phytophagous-miticidal mutant strain thereof can be applied to any plant or any part of any plant grown in any type of media used to grow plants (e.g., soil, vermiculite, shredded cardboard, and water) or applied to plants or the parts of plants grown aerially, such as orchids or staghorn ferns. The composition may for instance be applied by spraying, atomizing, vaporizing, scattering, dusting, watering, squirting, sprinkling, pouring or fumigating. As already indicated above, application may be carried out at any desired location where the plant of interest is positioned, such as agricultural, horticultural, forest, plantation, orchard, nursery, organically grown crops, turfgrass and urban environments.

Compositions of the present invention can be obtained by culturing *Streptomyces microflavus* NRRL B-50550 or mutants derived from it using conventional large-scale microbial fermentation processes, such as submerged fermentation, solid state fermentation or liquid surface culture, including the methods described, for example, in U.S. Patent No. 3,849,398, British Patent No. GB 1 507 193, Toshiko Kanzaki et al., Journal of Antibiotics, Ser. A, Vol. 15, No.2, Jun. 1961, pages 93 to 97, or Toru Ikeuchi et al., Journal of Antibiotics, (Sept. 1972), pages 548 to 550. Fermentation is configured to obtain high levels of live biomass, particularly spores, and desirable secondary metabolites in the fermentation vessels. Specific fermentation methods that are suitable for the strain of the present invention to achieve high levels of sporulation, cfu (colony forming units), and secondary metabolites are described in the Examples section.

The bacterial cells, spores and metabolites in culture broth resulting from fermentation (the "whole broth" or "fermentation broth") may be used directly or concentrated by conventional industrial methods, such as centrifugation, filtration, and evaporation, or processed into dry powder and granules by spray drying, drum drying and freeze drying, for example.

The terms "whole broth" and "fermentation broth," as used herein, refer to the culture broth resulting from fermentation (including the production of a culture broth that contains gougerotin in a concentration of at least about 1 g/L) before any downstream treatment. The whole broth encompasses the microorganism (e.g., *Streptomyces microflavus* NRRL B-50550 or a phytophagous-miticidal mutant strain thereof) and its component parts, unused raw substrates, and metabolites produced by the microorganism during fermentation. The term "broth concentrate," as used herein, refers to whole broth (fermentation broth) that has been concentrated by conventional industrial methods, as described above, but remains in liquid form. The term "fermentation solid," as used herein, refers to dried fermentation broth. The term "fermentation product," as used herein, refers to whole broth, broth concentrate and/or fermentation solids. Compositions of the present invention include fermentation products. In some embodiments, the concentrated fermentation broth is washed, for example, via a diafiltration process, to remove residual fermentation broth and metabolites.

In one embodiment, the fermentation broth or broth concentrate can be formulated into liquid suspension, liquid concentrate, or emulsion concentrate with the addition of stabilization agents, preservatives, adjuvants, and/or colorants.

In another embodiment, the fermentation broth or broth concentrate can be dried with or without the addition of carriers, inerts, or additives using conventional drying processes or methods such as spray drying, freeze drying, tray drying, fluidized-bed drying, drum drying, or evaporation.

In a further embodiment, the resulting dry products may be further processed, such as by milling or granulation, with or without the addition of inerts or additives to achieve specific particle sizes or physical formats or physical properties desirable for agricultural applications.

In addition to the use of whole broth or broth concentrate, cell-free preparations of fermentation broth of the novel variants and strains of *Streptomyces* of the present invention can be obtained by any means known in the art, such as extraction, centrifugation and/or filtration of fermentation broth. Those of skill in the art will appreciate that so-called cell-free preparations may not be devoid of cells but rather are largely cell-free or essentially cell-free, depending on the technique used (e.g., speed of centrifugation) to remove the cells. The resulting cell-free preparation may be dried and/or formulated with components that aid in its application. Concentration methods and drying techniques described above for fermentation broth are also applicable to cell-free preparations.

Compositions of the present invention may include formulation ingredients added to compositions comprising cells, cell-free preparations or metabolites to improve efficacy, stability, and physical properties, usability and/or to facilitate processing, packaging and end-use application. Such formulation ingredients may include carriers, inerts, stabilization agents, preservatives, nutrients, or physical property modifying agents, which may be added individually or in combination. In some embodiments, the carriers may include liquid materials such as water, oil, and other organic or inorganic solvents and solid materials such as minerals, polymers, or polymer complexes derived biologically or by chemical synthesis. In some embodiments, the ingredient is a binder, adjuvant, or adhesive that facilitates adherence of the composition to a plant part, such as leaves, seeds, or roots. *See,* for example, Taylor, A.G., et al., "Concepts and Technologies of Selected Seed Treatments" Annu. Rev. Phytopathol. 28: 321-339 (1990). The stabilization agents may include anti-caking agents, anti-oxidation agents, desiccants, protectants or preservatives. The nutrients may include carbon, nitrogen, and phosphors sources such as sugars, polysaccharides, oil, proteins, amino acids, fatty acids and phosphates. The physical property modifiers may include bulking agents, wetting agents, thickeners, pH modifiers, rheology modifiers, dispersants, adjuvants, surfactants, antifreeze agents or colorants. In some embodiments, the composition comprising cells, cell-free preparation or metabolites produced by fermentation can be used directly with or without water as the diluent without any other formulation preparation. In some embodiments, the formulation inerts are added after concentrating fermentation broth and during and/or after drying.

The present invention encompasses fermentation broths containing gougerotin at a concentration of at least about 1 g/L. In some embodiments such whole broth cultures come from gougerotin-producing strains of Streptomyces. In a particular embodiment, such gougerotin-producing strain is *Streptomyces microflavus, Streptomcyes puniceus* or *Streptomyces graminearus.* In yet another particular embodiment, such gougerotin-producing strain is Streptomyces microflavus NRRL B-50550 or phytophagous-miticidal mutants thereof. In yet another particular embodiment, such gougerotin-producing strain is *Streptomyces graminearus* CGMCC 4.506, deposited at China General Microbiological Culture Collection Center CGMCC. Fermentation broths containing at least about 1 g/L gougerotin may be obtained in several ways, such as fermentation optimization and/or mutagenesis of a parent gougerotin-producing strain in order to attain a mutant strain that produces higher levels of gougerotin than the parent strain.

Thus, the present invention also encompasses a method of producing a fermentation broth of a gougerotin producing *Streptomyces* strain, wherein the fermentation broth contains at least about 1 g/L gougerotin. The method comprises cultivating the *Streptomyces* strain in a culture medium that contains a digestible carbon source and a digestible nitrogen source under aerobic conditions, wherein the culture medium contains a precursor to gougerotin, such as cytosine; a nucleobase; and/or an amino acid at a concentration effective to achieve a gougerotin concentration of at least 1 g/L.

In some embodiments, the *Streptomyces* strain is cultivated in the culture medium until the culture medium contains gougerotin in a concentration of at least about 2 g/L, of at least about 3 g/L, of at least about 4 g/L, of at least about 5g/L, of at least about 6 g/L, of about at least 7 g/L or of at least about 8 g/L gougerotin.

In other embodiments, the *Streptomyces* strain is cultivated in the culture medium until the culture medium contains gougerotin in a concentration ranging from about 2 g/L to about 15 g/L gougerotin, meaning the fermentation broth contains gougerotin in a concentration ranging typically ranging from about 2 g/L to about 15 g/L gougerotin after completion of the fermentation. It is of course however possible that the fermentation broth has a gougerotin concentration that is higher than 15 g/L, in particular as a higher concentration of gougerotin leads to lower production costs. Thus, the upper concentration of gougerotin is by no means limited to 15 g/L but a fermentation broth with a higher gougerotin concentration is also encompassed by the present invention.

In this context it is noted that the amino acid that is added at a concentration effective to achieve a gougerotin concentration of at least 1 g/L is provided to the culture medium as a separate indivual component in a defined concentration and not part of a composition such as a yeast extract or a protein hydrolysate (for example, casein hydrolysate, soy flour hydrolysate, soy peptone, soy acid hydrolysate, to name only a few) in which amino acids may be present in a mixture with other compounds such as oligopeptides and partially hydrolyzed proteins. Thus, by "a concentration effective to achieve a gougerotin concentration of at least 1 g/L" in the fermentation broth is meant a concentration of an amino acid in the culture medium that is specifically chosen to provide such a gougerotin concentration. In some embodiments, the concentration effective to achieve the desired gougerotin concentration is a concentration of the amino acid in the culture medium of at least about 2 g/L. This "effective concentration" may thus be higher than 2 g/L and may, for example, range from about 2 g/L to about 15 g/L. The concentration may be about 3g/L, about 4 g/L, about 5 g/L, about 6 g/L, about 7 g/L, about 8 g/L, about 9 g/L, about 10 g/L, about 11 g/L, about 12 g/L, about 13 g/L, or about 14 g/L.

The amino acid may be any amino acid which provides for a concentration of gougerotin of at least about 1 g/L or a higher concentration such as at least about 2 g/L, of at least about 3 g/L, of at least about 4 g/L, of at least about 5g/L, of at least about 6 g/L, of method of any of claims 6 to 8. In some embodiments the amino acid is glycine, L-glutamic acid, L-glutamine, L-aspartic acid, or a mixture thereof. In some embodiments the culture medium contains glycine at a concentration of about 5g/L to about 15 g/L, whereas in other embodiments the culture medium contains glutamic acid in an initial concentration of about 5 g/L to about 15 g/L. It is also possible that the culture medium contains both glycine and L-glutamic acid (or L-glutamine) in a concentration of about 5 g/L to about 15 g/L.

Any carbon source that is digestible (and thus available) for Streptomcyes strains can be used in the method of producing a fermentation broth (or fermentation method) as described here. Examples of suitable carbon sources include glucose, fructose, mannose, galactose, sucrose, maltose, lactose, molasses, starch (as an example for a polysaccharide), dextrin, maltodextrin (as an example of an oligosaccharide) or glycerin, to name only a few. The total initial concentration of the carbon source (or sources) may be any concentration that provides a suitable growth of *Streptomyces* and production of the desired concentration of gougerotin and may be determined experimentally (determining the final concentration of gougerotin in the fermentation broth dependent from the concentration of the used carbon source(s)). The total initial carbon source concentration may, for example, be in the range of about 10 g/L to about 150 g/L, for example, about 10g/l, about 20 g/L, about 30 g/L, about 40 g/L, about 50 g/L, about 60 g/L, about 70 g/L, about 80 g/L, about 90 g/L, about 100 g/L, about 110 g/L or about 120 g/L. In some embodiments, the carbon source might be a mixture of two or more carbon sources, for example, a mixture of glucose with a polysaccharide such as starch, a mixture of glucose and an oligosaccharide such as dextrin or maltodextrin or a mixture of glucose, starch and dextrin. In some embodiments the culture medium contains as carbon source a mixture of glucose and an oligosaccharide. The oligosaccharide may be maltodextrin or dextrin. In such embodiments, the initial maltodextrin concentration in the culture medium may be about 50 g/L to about 100 g/L or about 60 g/L to about 80 g/L.The initial glucose concentration in the culture medium may be about 20 g/L to about 80 g/L, for example, about 30 g/L, about 40 g/L, about 50 g/L, about 60 g/L or about 70 g/L. In other embodiments in which glucose is used as carbon source with maltodextrin or dextrin, the glucose concentration may be about 20 g/L to 60 g/L or about 30 g/L to about 50 g/L.

Any nitrogen source that is digestable can be used in the fermentation process described here. The nitrogen source can be a single source or a mixture of sources. In illustrative embodiments the nitrogen source is (at least partially) selected from the group consisting of soy peptone, soy acid hydrolysate, soy flour hydrolysate, casein hydrolysate, yeast extract, and mixtures thereof. The total initial concentration of the nitrogen source(s) may be any concentration that provides a suitable growth of *Streptomyces* and production of the desired concentration of gougerotin and may be determined experimentally. Suitable total concentrations in the culture medium may, for example, be in the range of about 10 g/L to about 60 g/L, for example, about 20 g/L, about 30 g/L, about 40 g/L, about 50 g/L. In illustrative embodiments, the nitrogen source may be a mixture of casein hydrolysate and soy flour hydrate or a mixture of yeast extract and soy acid hydrolysate, wherein for example the yeast extract is used in the culture medium in a concentration (or amount) of 10 g/L and the soy acid hydrolysate is used in a concentration/amount of 20.0 g/L

The culture medium can further contain a calcium source such as calcium chloride, or calcium carbonate. If present, the culture medium may contain a calcium source such as calcium carbonate in an initial concentration of about 1 g/L to 3g/L.

In this context, it is noted that concentrations of all ingredients of the culture medium are given as concentration at the beginning of the fermentation (initial concentrations) unless indicated otherwise. The concentrations are based on the post inoculation volume that is used for the fermentation. The initial concentrations as given here can either be maintained during the fermentation by continuous nutrient feeding or, alternatively, the ingredients (carbon source, nitrogen source, amino acid) can be added only at the beginning of the fermentation. However, the pH of the culture medium/fermentation broth is typically continuously monitored and controlled by addition of a suitable acid (such as sulfuric acid or citric acid) and/or of a suitable base (such as sodium hydroxide or ammoniak solution or potassium hydroxide). An appropriate pH can be determined empirically. In typical embodiments the pH of the culture medium/fermentation broth is in range of 6.5 to 7.5, for example, 6.8 to 7.0. Also process parameters such as temperature and aeration rate are usually controlled over the course of fermentation process. Since the cultivation of the *Streptomyces* strain is carried out under aerobic conditions, the fermentation broth is typically aerated with air, oxygen enriched air or if necessary, pure oxygen. The temperature is usually chosen to be within a range of 20°C to 30°C, however higher temperatures are also contemplated herein. Standard fermentation reagents such as antifoam agents may also be added continuously. The production of the fermentation broth can be carried out using conventional large-scale microbial fermentation processes, such as submerged fermentation, solid state fermentation or liquid surface culture, including the methods described, for example, in U.S. Patent No. 3,849,398, British Patent No. GB 1 507 193, Toshiko Kanzaki et al., Journal of Antibiotics, Ser. A, Vol. 15, No.2, Jun. 1961, pages 93 to 97, or Toru Ikeuchi et al., Journal of Antibiotics, (Sept. 1972), pages 548 to 550.

Any gougerotin producing *Streptomcyes* strain can be used for producing the gougerotin containing fermentation broth disclosed herein. In illustrative embodiments the *Streptomcyes* strain is a *Streptomyces microflavus* strain or a *Streptomcyes puniceus* strain. The *Streptomyces microflavus* strain may, for example, be *Streptomyces microflavus* strain NRRL B-50550 or a phytophagous-miticidal mutant strain derived therefrom. In addition, parent bacterial strains, such as various Streptomycetes (including, but not limited to, *Streptomyces microflavus* and *Streptomcyes puniceus*) and Bacilli, capable of producing gougerotin, even at low levels, may be mutagenized for enhanced gougerotin production. Example 14 describes one way to produce such mutants and resulting fermentation broths containing at least 1 g/L gougerotin.

Selection of specific carbon and nitrogen sources and other nutrients during fermentation may be used to optimize the production of gougerotin. Suitable carbon sources for enhancing gougerotin production are starch, maltodextrin, dextrin, sugars and glucose. In a specific embodiment a combination of glucose and an oligosaccharide is used as the carbon source and/or procures. Suitable nitrogen sources for enhancing gougerotin production are soy protein hydrolysate, casein hydrolysate, soy peptone, yeast extract, and other nitrogen sources that are less nutrient rich. Other suitable nitrogen sources include amino acids and/or precursors to gougerotin such as glycine, glutamic acid, including L-glutamic acid, aspartic acid, including L-aspartic acid and cytosine. Cytosine may be added as part of a media component that has a high concentration of cytosine, such as a yeast extract having high nucleobase content. Examples of fermentation media capable of producing a fermentation broth having an increased level of gougerotin are provided in Examples 11, 12 and 13.

In some embodiments the compositions of the present invention are used to treat a wide variety of agricultural and/or horticultural crops, including those grown for seed, produce, landscaping and those grown for seed production. Representative plants that can be treated using the compositions of the present invention include but are not limited to the following: brassica, bulb vegetables, cereal grains, citrus, cotton, cucurbits, fruiting vegetables, leafy vegetables, legumes, oil seed crops, peanut, pome fruit, root vegetables, tuber vegetables, corm vegetables, stone fruit, tobacco, strawberry and other berries, and various ornamentals.

The compositions of the present invention may be administered as a foliar spray, as a soil treatment, and/or as a seed treatment/dressing. When used as a foliar treatment, in one embodiment, about 1/16 to about 5 gallons of whole broth are applied per acre. When used as a soil treatment, in one embodiment, about 1 to about 5 gallons of whole broth are applied per acre. When used for seed treatment about 1/32 to about 1/4 gallons of whole broth are applied per acre. For seed treatment, the end-use formulation contains at least 1 x 10⁸ colony forming units per gram.

In some embodiments, application of the compositions of the present invention to plants, plant parts or plant loci is preceded by identification of a locus in need of treatment.

### DEPOSIT INFORMATION

A sample of the *Streptomyces microflavus* strain of the invention has been deposited with the Agricultural Research Service Culture Collection located at the National Center for Agricultural Utilization Research, Agricultural Research Service, U.S. Department of Agriculture, 1815 North University Street, Peoria, IL 61604 under the Budapest Treaty and has been assigned the following depository designation: NRRL B-50550.

The following examples are given for purely illustrative and non-limiting purposes of the present invention.

### EXAMPLES

### Example 1 - Selection of Streptomyces microflavus NRRL B-50550

Strains were taken from an internal collection of strains and initial screening tests were conducted to determine efficacy of potential candidates strain against two-spotted spider mites ("TSSM"), which are a model organism commonly used to screen for general miticidal activity. Microorganisms were selected initially for properties that favor laboratory or artificial cultivation, such as variants that grow rapidly on an agar plate. Culture stocks of the selected strains were grown in suitable media for the respective strain, such as the Medium 1 and Medium 2 described in Example 2. The resulting fermentation products (whole broths) were diluted to a 25% solution using water and 0.03% of the surfactant BREAK-THRU FIRST CHOICE^{®}. Thereafter, 8 ml of the diluted fermentation products were applied to run-off to the top and bottom of lima bean leaves of two plants (the lima bean plants were 1 to 1.5 weeks old). After such treatment, plants were infested on the same day with 50-100 TSSM and left in the greenhouse for five days. On the fifth day plants were assessed for presence of mites and eggs on a scale of 1 to 4. The miticide Avid^{®} (Syngenta) was used as positive control. For mites and eggs, 1 indicates 100% mortality, 1.5 indicates 90% to 95% mortality, 2.0 represents 75% to 90% mortality; 2.5 represents 40% to 55% mortality; 3.0 represents 20% to 35% mortality and 4.0 represents 0% to 10% mortality. Besides NRRL B-50550, other *Streptomcyes* strains and some Bacillus strains were found to be active against mites.

For further selection, amongst other activities, the UV stability and translaminar activity of the screened strains was examined since an acaracide should be stable to UV light and possess translaminar activity in order to be effective in field applications.

For assessment of the UV stability the above-described 25 % dilutions of the fermentation products were sprayed on the upper surface of lima bean plants. After such treatment, plants were infested on the same day with 50-100 TSSM, exposed to UV light for 24 hrs and left in the greenhouse for five days. The mites were confined to the adaxial (upper) surface of the leaves by means of a Vaseline ring which was applied to the leaf and served as an impassable boundary to the mites. On the fifth day plants were assessed for presence of mites and eggs on a scale of 1 to 4, as described above. The miticides Avid^{®} (Syngenta) and Oberon^{®} (Bayer CropScience AG) were used as controls. Results are shown in Fig. 1. The fermentation product of the strain NRRL B-50550 showed the best UV stability of all strains tested.

For assessment of the translaminar activity the strains were cultured as described above and the resulting whole broth was diluted using water and 0.35% surfactant and applied to run-off to the lower surface of lima bean leaves on two plants. The upper surface of the treated leaves was infested one day after treatment with 50-100 TSSM, which were placed on the upper surface of the leaves and contained using a Vaseline ring/physical barrier as described above. On the sixth day plants were assessed for presence of mites and eggs on the above-described scale of 1 to 4. The miticides Avid^{®} (Syngenta) and Oberon^{®} (Bayer CropScience AG) were used as controls. Results are shown in Fig. 2. The fermentation product of the strain NRRL B-50550 showed the best translaminar activity of all strains tested.

### Example 2 - Activity against Spider Mites

Further tests were conducted to more closely determine the efficacy of *Streptomyces microflavus* NRRL B-50550 against two-spotted spider mites ("TSSM"). Culture stocks of Streptomyces *microflavus* NRRL B-50550 were grown in 1 L shake flasks in Medium 1 or Medium 2 at 28°C for 5 days. Medium 1 was composed of 2.0 % starch, 1.0% dextrose, 0.5% yeast extract, 0.5% casein hydrolysate and 0.1% CaCO₃. Medium 2 was composed of 2% ProFlo cotton seed meal, 2% malt extract, 0.6% KH₂PO₄ and 0.48% K₂HPO₄. The resulting fermentation products were diluted to a 25% solution using water and 0.03% surfactant BREAK-THRU FIRST CHOICE^{®} and applied to run-off to the top and bottom of lima bean leaves of two plants. After such treatment, plants were infested on the same day with 50-100 TSSM and left in the greenhouse for five days. On the sixth day plants were assessed for presence of mites and eggs on a scale of 1 to 4. The miticide Avid^{®} (Syngenta) was used as positive control. For mites and eggs, 1 indicates 100% mortality, 1.5 indicates 90% to 95% mortality, 2.0 represents 75% to 90% mortality; 2.5 represents 40% to 55% mortality; 3.0 represents 20% to 35% mortality and 4.0 represents 0% to 10% mortality. Results are shown in Table 1 below. Both fermentation products of *Streptomyces microflavus* NRRL B-50550 resulted in a mortality of mites of 90% or greater.

**Table 1**

| Fermentation Product | Mites | Eggs |
|---|---|---|
| NRRL B-50550 Medium 1 | 1.25 | 1.00 |
| NRRL B-50550 Medium 2 | 1.50 | 1.50 |
| Positive Control (AVID^{®} EC - 5.7 ppm) | 1.00 | 1.00 |
| Untreated Control | 3.75 | 4.00 |

Field trials against Pacific spider mite in almond, Pacific spider mite in grapes, and two-spotted spider mite in strawberry, confirmed the above greenhouse results. Results of field trials against Pacific spider *mite* in almonds are reported in Tables 2-4, below. The miticide AGRI-MEK^{®} (Syngenta) was used as positive control. Shake flasks containing Medium 1 were inoculated with frozen cultures of NRRL-50550 and grown 1-2 days at 28-30 °C. The resulting fermentation product was used to seed a 20-L bioreactor containing the following media: 6.0% starch, 3.0% dextrose, 1.5% yeast extract and 1.5% casein hydrolysate and 0.3% calcium carbonate. This medium was fermented at between 28 °C for 7 days. The resulting whole broth was used to create a freeze dried powder ("FDP") that was mixed with an adjuvant, BREAK-THRU FIRST CHOICE^{®}, at 0.03% and then used in the trial.

**Table 2 - Activity against Adult Mites**

| No. Adult Mites/Leaf | 0 DAT | 3 DAT | 7 DAT | 14 DAT |
|---|---|---|---|---|
| Untreated | 9.3 | 8.8 | 10.5 | 5.8 |
| NRRL B-50550 FDP 0.63 lb/acre | 15.0 | 0.8 | 0.0 | 0.0 |
| NRRL B-50550 FDP 0.125 lb/acre | 13.5 | 1.3 | 0.8 | 0.3 |
| NRRL B-50550 FDP 2.5 lb/acre | 15.0 | 0.8 | 0.0 | 0.0 |
| NRRL B-50550 FDP 5 lb/acre | 16.8 | 0.0 | 0.3 | 0.0 |
| Standard (AGRI-MEK 0.15 EC at 16 fl. oz./acre) | 7.0 | 0.0 | 0.0 | 0.0 |

**Table 3 - Activity against Juvenile Mites**

| No. Juvenile Mites/Leaf | 0 DAT | 3 DAT | 7 DAT | 14 DAT |
|---|---|---|---|---|
| Untreated | 23.8 | 24.3 | 29.8 | 12.5 |
| NRRL B-50550 FDP 0.63 lb/acre | 43.0 | 3.0 | 1.8 | 0.0 |
| NRRL B-50550 FDP 0.125 lb/acre | 31.5 | 2.0 | 1.3 | 0.0 |
| NRRL B-50550 FDP 2.5 lb/acre | 41.8 | 2.0 | 0.8 | 0.0 |
| NRRL B-50550 FDP 5 lb/acre | 39.3 | 0.8 | 0.3 | 0.0 |
| Standard (AGRI-MEK 0.15 EC at 16 fl. oz./acre) | 37.5 | 0.5 | 1.0 | 0.3 |

**Table 4 - Activity against Mite Eggs**

| No. Mite Eggs/Leaf | 0 DAT | 3 DAT | 7 DAT | 14 DAT |
|---|---|---|---|---|
| Untreated | 26.8 | 21.0 | 19.8 | 9.5 |
| NRRL-50550 FDP 0.63 lb/acre | 23.5 | 4.8 | 5.5 | 0.0 |
| NRRL-50550 FDP 0.125 lb/acre | 16.3 | 3.0 | 2.3 | 0.5 |
| NRRL-50550 FDP 2.5 lb/acre | 29.0 | 3.3 | 2.8 | 0.0 |
| NRRL-50550 FDP 5 lb/acre | 33.8 | 5.0 | 3.3 | 0.8 |
| Standard (AGRI-MEK 0.15 EC at 16 fl. oz./acre) | 22.3 | 5.8 | 1.3 | 0.3 |

### Example 3 - Field Activity against Citrus Mite

Field trials were conducted to determine efficacy of NRRL B-50550 against citrus rust mites *(Phyllocoptruta oleivora*) on Valencia oranges. Shake flasks containing Medium 1 (see Example 2) were inoculated with frozen cultures of NRRL B-50550 and grown 1-2 days at 20-30 °C. This was repeated. The resulting fermentation product was used to seed a 20-L bioreactor containing the following media: 6.0% starch, 3.0% dextrose, 1.5% yeast extract, 2.0% soy acid hydrolysate, 0.6% glycine, and 0.2% calcium carbonate. This medium was fermented at between 28 °C for 8 days. The resulting whole broth was used to create a freeze dried powder ("FDP") used in the following trials. The freeze dried powder was diluted in water and applied at 100 gal/acre at the rates shown in Table 5 below. The miticide Envidor^{®} (Bayer Crop Science, Germany) was used as positive control. In treatments 1-3, the BREAK-THRU FIRST CHOICE adjuvant (see above) was added at 0.66% v/v. The fermentation product applied at a rate of 0.625-lb/A showed a better miticidal activity then then Envidor^{®} applied at a rate of 16-fl oz/A.

**Table 5**

| | Treatment | Rate | No. Mites/cm² Fruit |
|---|---|---|---|
| 1. | NRRL B-50550 FDP | 0.625-lb/A | 0.29 |
| 2. | NRRL B-50550 FDP | 1.25-lb/A | 1.43 |
| 3. | NRRL B-50550 FDP | 2.5-lb/A | 0.78 |
| 4. | NRRL B-50550 + 435 Oil | 2.5-lb/A + 5-gal/A | 0.76 |
| 5. | Envidor 2SC | 16-fl oz/A | 0.41 |
| 6. | Untreated Check | - - | 13.09 |

### Example 4 - Activity against other mites

Studies have shown that NRRL B-50550 is active against various other mites including eriophyid (russet) mites and broad mites. Fermentation broth was prepared as it was for the field trials described in Example 2. The resulting fermentation broth was diluted to various concentrations using water and 0.35% surfactant and applied to run-off to the top and bottom of lima bean leaves on two plants. Plants were infested on the day of treatment and assessed for presence of mites on the scale described above 6 days after treatment. A score of four indicated no control and presence of at least 100 russet mites at time of assessment. The miticide Avid^{®} was used as positive control.

**Table 6**

| Treatment | Rating -new leaves | Rating - old leaves |
|---|---|---|
| NRRL B-50550 WB 12.50% | 1.58 | 1.50 |
| NRRL B-50550 WB 6.25% | 1.75 | 1.92 |
| NRRL B-50550 WB 3.12% | 2.42 | 2.67 |
| NRRL B-50550 WB 1.56% | 2.75 | 3.17 |
| Untreated | 4.00 | 4.00 |
| Avid (EC) 1% | 1.00 | 1.00 |

### Example 5 - Residual Activity

Other studies revealed that NRRL B-50550 has residual activity. Shake flasks containing Medium 1 of Example 2 were inoculated with Luria broth based cultures of NRRL B-50550 (which had been inoculated with a frozen culture of NRRL B-50550) and grown 1-2 days at 28 °C. The resulting fermentation product was used to seed a 20-L bioreactor containing the following media: 8.0% dextrose, 1.5% yeast extract, 1.5% casein hydrolysate and 0.1% calcium carbonate. This medium was fermented at between 28 °C for 7-8 days. The resulting fermentation product was diluted to 3.13% solution using water and 0.35% surfactant and applied to run-off to the top and bottom of lima bean leaves on two plants. Plants were infested six days after such treatment with 50-100 TSSM and assessed for presence of mites and eggs on the scale described above 12 days after treatment. The miticide Avid^{®} was used as positive control. Results are shown in Table 7 below.

**Table 7**

| Fermentation Product | Mites | Eggs |
|---|---|---|
| NRRL-50550 WB 3.13% | 1.12 | 1.31 |
| Positive Control (AVID^{®} - 0.4 ul/10ml) | 1.00 | 1.00 |
| Untreated Control | 4.00 | 4.00 |

### Example 6 - Translaminar activity

Studies were conducted to determine whether NRRL-50550 has translaminar activity. Whole broth was prepared as described in Example 5. The resulting whole broth was diluted using water and 0.35% surfactant and applied to run-off to the lower surface of lima bean leaves on two plants. The upper surface of the treated leaves was infested one day after treatment with 50-100 TSSM, which were placed on the upper surface of the leaves and contained using a Vaseline ring/physical barrier placed on the upper surface of the leaves. Plants were assessed for presence of mites and eggs on the scale described above five days after treatment. Results are shown in Table 8 below.

**Table 8**

| Treatment | Mites | Eggs |
|---|---|---|
| NRRL B-50550 WB 12.5% | 1.00 | 1.19 |
| NRRL B-50550 WB 6.25% | 1.51 | 1.73 |
| NRRL B-50550 WB 3.12% | 2.50 | 2.44 |
| NRRL B-50550 WB 1.56% | 2.12 | 2.19 |
| Positive Control AVID^{®} - 0.8 ul/10ml) | 1.46 | 1.30 |
| Untreated Control | 3.50 | 3.62 |

### Example 7 - Ovicidal Activity

NRRL B-50550 was tested for ovicidal activity as follows. Whole broth was prepared as described in Example 5. Two lima bean plants were preinfested with TSSM eggs by allowing adult female mites to oviposit on the leaf surface for 48 hours prior to treatment. Plants were then treated with various dilutions of whole broth. Plants were assessed five days after treatment. The number of live and dead eggs present in each treatment and control are shown in Table 9 below.

**Table 9**

| Treatment | Live Eggs | Dead Eggs |
|---|---|---|
| NRRL B-50550 (6.25%) | 1.00 | 32.75 |
| NRRL B-50550 (3.12%) | 0.50 | 18.25 |
| NRRL B-50550 (1.56%) | 1.00 | 20.50 |
| Positive Control (OBERON SC - spiromesifen 4 fl oz/100gal) | 1.50 | 75.00 |
| Untreated Control | 24.00 | 2.00 |

### Example 8 - Drench Activity

Drench activity of NRRL B-50550 was studied using lima beans grown in sand. Whole broth was prepared as described in Example 3. Two applications of 10 ml each of a 12.5% dilution of whole broth were applied to the sand. Plants were watered carefully to prevent leaching of whole broth from the bottom of the pot. Applications were made at four days after planting and at five days after planting. Lower leaves were infested with motile TSSM three days after treatment two. The upper leaf trifoliate was infested nine days after lower leaves were infested. Assessments were made on lower leaves at 4, 5, 8 and 11 days after infestation. Assessments on upper leaves were conducted at two days after infestation. Results, based on the scoring system described in Example 2, are shown in Table 10 below.

**Table 10**

| | Mites | Eggs | % upper leaf surface stippled |
|---|---|---|---|
| NRRL-50550 - 1st Assessment [Lower Leaves] | 1.83 | 1.43 | 7.00 |
| NRRL-50550 - 2^{nd} Assessment [Lower Leaves] | 1.33 | 1.5 | 5.00 |
| NRRL-50550 - 3rd Assessment [Lower Leaves] | 1.05 | 1.05 | 2.75 |
| NRRL-50550 - 4thAssessment [Lower Leaves] | 1.83 | 1.38 | 4.5 |
| NRRL-50550 - 1^{st} Assessment [Upper Leaves] | 1.93 | 1.43 | 4.25 |
| Untreated Control - 1^{st} Assessment [Lower Leaves] | 3.63 | 3.45 | 23.8 |
| Untreated Control -2^{nd} Assessment [Lower Leaves] | 3.88 | 4 | 25 |
| Untreated Control - 3^{rd} Assessment [Lower Leaves] | 4 | 4 | 52.5 |
| Untreated Control - 4^{th} Assessment [Lower Leaves] | 4 | 4 | 80 |
| Untreated Control - 1^{st} Assessment [Upper Leaves] | 4 | 4 | 77.5 |

### Example 9 - Activity against Fungal Phytopathogens

NRRL-50550 was tested for activity against various plant fungal pathogens. It was found to be active against both wheat leaf rust and cucumber powdery mildew. Shake flasks containing Medium 1 were inoculated with frozen cultures of NRRL B-50550 and grown 1-2 days at 20-30 °C. The resulting fermentation product was used to seed a 20-L bioreactor containing similar media and grown 1-2 days at 28 °C. The resulting fermentation product was, in turn, used to seed a 200 L fermentor containing the following media: 7.0% starch, 3.0% dextrose, 1.5% yeast extract, 2.0% soy acid hydrolysate, 0.8% glycine, and 0.2% calcium carbonate. This medium was fermented at between 26 °C for 8 days. Six-day old wheat seedlings were treated with NRRL-50550 whole broth prepared at various dilutions with 0.03% adjuvant (BREAK-THRU FIRST CHOICE^{®}) shown in Table 11 below by covering both leaf surfaces with whole broth and allowing to dry. Seedlings were inoculated with a wheat leaf rust suspension one day after such treatment. Plants were rated about a week after treatment using the following scale on a 0-100% control, where 0% is no control and 100% is perfect control.

**Table 11**

| Treatment | Rate | Control |
|---|---|---|
| NRRL B-50550 WB | 20% | 98.7 |
| NRRL B-50550 WB | 5% | 95.0 |
| NRRL B-50550 WB | 1.25% | 50.0 |
| NRRL B-50550 WB | 0.3125% | 0.0 |
| NRRL B-50550 Supernatant | 20% | 95.0 |
| NRRL B-50550 Supernatant | 5% | 66.7 |
| NRRL B-50550 Supernatant | 1.25% | 0.0 |
| NRRL B-50550 Supernatant | 0.3125% | 0.0 |
| NRRL B -50550 Cell Extract | 20% | 50.0 |
| NRRL B-50550 Cell Extract | 5% | 50.0 |
| NRRL B-50550 Cell Extract | 1.25% | 0.0 |
| NRRL B-50550 Cell Extract | 0.3125% | 0.0 |
| Untreated Check | | 0.0 |
| Adjuvant Check | | 0.0 |

In addition, NRRL-50550 showed activity against cucumber powdery mildew when whole broth was applied on the lower leaf surface and the pathogen was applied on the upper leaf surface.

### Example 10 - Corn Rootworm Activity

Tests were conducted to determine efficacy of NRRL B-50550 against corn rootworm. NRRL B-50550 whole broth was prepared in Medium 1 or Medium 2, as described in Example 2. NRRL B-50550 whole broth was diluted and fed to larvae of western spotted cucumber beetle (*Diabrotica undecimpunctata*) in a diet-based assay conducted in a microtiter plate. Activity was assessed and rated on a scale of 1 to 4, as described in Example 2. The termiticide/insecticide Termidor^{®} SC (BASF) was used as positive control. Results are shown in Table 12. NRRL B-50550 showed the same insecticidal activity as the insecticide Termidor^{®} SC.

**Table 12**

| Treatment | Dosage | Rating |
|---|---|---|
| NRRL B-50550 Media 1 | 1.56% | 1.0 |
| NRRL B-50550 Media 1 | 100% | 1.0 |
| NRRL B-50550 Media 1 | 25% | 1.0 |
| NRRL B-50550 Media 1 | 6.25% | 1.0 |
| NRRL B-50550 Media 2 | 1.56% | 4.0 |
| NRRL B-50550 Media 2 | 100% | 1.0 |
| NRRL-50550 Media 2 | 25% | 1.0 |
| NRRL-50550 Media 2 | 6.25% | 1.0 |
| Termidor SC | 1.56% | 1.0 |
| Termidor SC | 100.0% | 1.0 |
| Termidor SC | 25.0% | 1.0 |
| Untreated | | 4.0 |

### Example 11 - Fermentation Product Containing Increased Levels of Gougerotin

Fermentation was conducted to optimize gougerotin production and miticidal activity of NRRLB-50550. A primary seed culture was prepared as described in Example 1 using a media composed of 10.0 g/L starch, 15.0 g/L glucose, 10.0 g/L yeast extract, 10.0 g/L casein hydrolysate (or 10.0 g/l *soy* peptone) and 2.0 g/L CaCO₃ in 2L shake flasks at 20-30 °C. When there was abundant mycelial growth in the shake flasks, after about 1-2 days, the contents were transferred to fresh media (same as above, with 0.1 % antifoam) and grown in a 400 L fermentor at 20-30 °C. When there was abundant mycelial growth, after about 20-30 hours, the contents were transferred to a 3000 L fermentor and grown for 160-200 hours at 20-30 °C in media composed of 80.0 g/L (8.0%) Maltodextrin , 30.0 g/L (3.0%) glucose, 15.0 g/L (1.5%) yeast extract, 20.0 g/L (2.0%) soy acid hydrolysate, 10.0 g/L (1.0%) glycine and 2.0 g/L (0.2%) calcium carbonate and 2.0 ml/L antifoam.

**Yield and Normalized Gougerotin Productivity**

| | **Harvest Titer (mg/g)** | **Harvest Weight (kg)** | **Total Gougerotin (kg)** | **Target Volume (L)** | **Normalized Volumetric Titer (g/L)** |
|---|---|---|---|---|---|
| First 3000L Fermentation | 5.58 | 3397 | 18.96 | 3000 | **6.32** |
| Second 3000L Fermentation | 5.55 | 3511 | 19.49 | 3000 | **6.50** |

Using the first 3000 L fermentation as an example, the yield of gougerotin in the fermentor is calculated as follows. 3397 kg x 5.58 mg/g Fermentation broth = 18955 g gougerotin = 18.96 kg. The initial weight in the fermentor was 3496 kg (3256 kg Medium + 240 kg Seed), which resulted in a final volume more than the target volume 3000L. Since the target volume 3000 L is the basis for calculating the amount of all ingredients in the production medium, the normalized volumetric productivity is: 18955 g/3000L = 6.32 g/L. This gougerotin concentration was similar to the 7 g/L achieved in a 20 L fermentation conducted using the same media as described above, with the final fermentation step and media containing glycine (as amino acid).

### Example 12 - Fermentation Product Containing Increased Levels of Gougerotin

Fermentation was conducted to optimize gougerotin production and miticidal activity of NRRLB-50550. A primary seed culture was prepared as described in Example 1 using a media composed of 10.0 g/L starch, 15.0 g/L glucose, 10.0 g/L yeast extract, 10.0 g/L casein hydrolysate (or 10.0 g/l soy peptone) and 2.0 g/L CaCO₃ in 1L shake flasks at 20-30 °C. When there was abundant mycelial growth in the shake flasks, after about 1-2 days, the contents were transferred to fresh media (same as above, with 0.1 % antifoam) and grown in 1 L shake flasks at 20-30 °C. When there was abundant mycelial growth, after about 20-30 hours, the contents were transferred to a 20 L fermentor and grown for 160-200 hours at 20-30 °C in media composed of 60.0 g/L (8.0%) starch, 30.0 g/L (3.0%) dextrose, 15.0 g/L (1.5%) yeast extract, 20.0 g/L (2.0%) soy acid hydrolysate, 12.0 g/L (1.0%) L-glutamic acid and 2.0 g/L (0.2%) calcium carbonate and 2.0 ml/L antifoam.

This gougerotin concentration using L-glutamic acid as amino acid in this fermentation was 3.1 g/L.

### Example 13 - Fermentation Product Containing Increased Levels of Gougerotin

Applicant tested gougerotin production by *Streptomyces microflavus* B-50550 in media to which cytosine, thymine and/or uracil were added. Specifically, Streptomyces microflavus B-50550 was grown in a media composed of 20.0 g/L maltodextrin, 10.0 g/L glucose, 5.0 g/L yeast extract, 6.0 g/L soy protein acid hydrolysate, 2.0 g/L glycine, 1.0 g/L CaCO₃ and cytosine, uracil and/or thymine, each at a concentration of 0 or 0.50 g/L, in 2L shake flasks at 20-30 C for 6 days. Results are shown in the table below.

| **Run** | **Cytosine (g/L)** | **Uracil (g/L)** | **Thymine (g/L)** | **Gougerotin at Day 4 (g/L)** | **Gougerotin at Day 6 (g/L)** |
|---|---|---|---|---|---|
| 1 | 0.50 | 0.50 | 0 | 1.6 | 2.4 |
| 2 | 0.50 | 0 | 0.50 | 1.4 | 2.2 |
| 3 | 0.50 | 0.50 | 0.50 | 1.2 | 2.1 |
| 4 | 0 | 0.50 | 0.50 | 1.3 | 1.7 |
| 5 | 0.50 | 0 | 0 | 1.6 | 2.4 |
| 6 | 0 | 0.50 | 0 | 1.4 | 2.0 |
| 7 | 0 | 0 | 0.50 | 1.1 | 1.4 |
| 8 | 0 | 0 | 0 | 1.2 | 1.5 |

### Example 14 - Gougerotin-Overproducing Mutants

With the goal of increasing gougerotin production and bioactivity, mutants were created from the parent strain *Streptomyces microflavus* NRRL No. B-50550 through an antibiotic-resistant mutant screening program in which libraries of mutants resistant to individual antibiotics (gentamicin, rifampicin, streptomycin, paromomycin or tobramycin) were produced. See Okamoto-Hosoya, Y., et al., The Journal of Antibiotics 43(12) Dec 2000. The parent strain was subjected to mutagenesis using N-methyl-N'-nitro-N-nitrosoguanidine ("NTG") and then resulting antibiotic resistant mutants selected and screened. A detailed description of creation and screening of mutant libraries from which gougerotin-overproducing strains were selected for further development is described below.

Spore suspensions of Streptomyces microflavus B-50550 were prepared from soy flour maltose (SFM) agar plates containing B-50550 grown for approximately 14 days or to sporulation and stored at -80°C in 20% glycerol. NTG, dissolved in suitable buffer, was added to the spore suspensions in an amount suitable to obtain 50% kill (0.5 mg/ml at pH8.5 slowly shaken for 1 hour at 37°C). NTG-treated spore suspensions were then plated onto GYM (glucose 4g/L, yeast extract 4g/L, malt extract 10g/L, and agar 12g/L) supplemented with the following concentrations of antibiotics. See table below.

| ANTIBIOTIC | 1X | 2X | 5X | 10X | 20X |
|---|---|---|---|---|---|
| STREPTOMYCIN SO4 | 10mg/L | 20mg/L | 50mg/L | 100mg/L | 200mg/L |
| RIFAMPICIN (Fresh) | 3.5mg/L | 7mg/L | 17.5mg/L | 35mg/L | 70mg/L |
| PAROMOMYCIN SO4 | 1 mg/L | 2mg/L | 5 mg/L | 10mg/L | 20mg/L |
| TOBRAMYCIN SO4 | 4.5mg/L | 9mg/L | 22.5mg/L | 45mg/L | 90mg/L |
| GENTAMYCIN SO4 | 5.5mg/L | 11mg/L | 27.5mg/L | 55mg/L | 11mg/L |

See Kieser, T., et al., Practical Streptomyces Genetics, Ch. 5 John Ines Centre Norwich Research Park, England (2000), pp. 99-107. Approximately 350 individual antibiotic-resistant colonies were isolated, purified, and screened as described below.

Each isolate removed from GYM antibiotic plates was re-plated onto SFM agar plates. Agar plugs containing antibiotic-resistant bacteria were used to inoculate 24-well blocks containing 2.5 mls of seed media. Bacteria in these inoculated blocks were grown for 3 days and the resulting culture broth used to inoculate 24-well blocks containing production media. Bacteria in production blocks were grown for seven days at 28 C. Each well in the seed blocks contained Trypticase Soy Broth (TSB) (Per liter of DI H20: 17g Bacto Tryptone (Pancreatic Digest of Casein), 3g Bacto Soytone (Pancreatic Digest of Soybean Meal), 2.5g Dextrose, 5g NaCl, 2.5g Dipotassium Phosphate) and in the production blocks contained Medium 2 of Example 2 (Proflo 20g/L, malt extract 20g/L, KH₂PO₄ monobasic 6g/L, K₂HPO₄ dibasic 4.8g/L).

The whole broth from each well of the production block was tested for gougerotin production as follows using analytical HPLC chromatography. 2.4 ml water was added to each well of the production block. Blocks were vortexed and centrifuged. 0.8 ml supernatant was transferred to an extraction block containing 4 ml of water per well. 3.2 ml water was added to the cell pellet in each well of the production block and the block vortexed and centrifuged again. This 3.2 ml of wash water was then added to the appropriate well of each extraction block. The aqueous extracts in the extraction block were then assayed for gougerotin content using analytical HPLC chromatography. Specifically, a sample was injected onto a Cogent Diamond hydride column (100A, 4um, 150 x 4.6mm) fitted with a Diamond Hydride guard column. The column was eluted with a 30 minute Acetonitrile/NH40AC gradient (see below). The flow rate was 1ml/min. Gougerotin was detected at 254 nm. Gougerotin elutes as a single peak with an approximate retention time of 19 minutes. Top over-producing mutants were confirmed by re-growing in both 24 well blocks and 250 ml flasks to confirm gougerotin levels. Once confirmed some isolates were then subjected to at least one more round of mutagenesis and antibiotic-resistance screening. Each subsequent round of mutagenesis coupled with antibiotic-screening was performed using the remaining antibiotics to which an isolate derived in the previous round had not developed resistance. Small (1.2X) increases in gougerotin production were found after a single round of screening, and subsequent rounds lead to greater increases from isolates generated from the same original low level overproducer. See following figure.

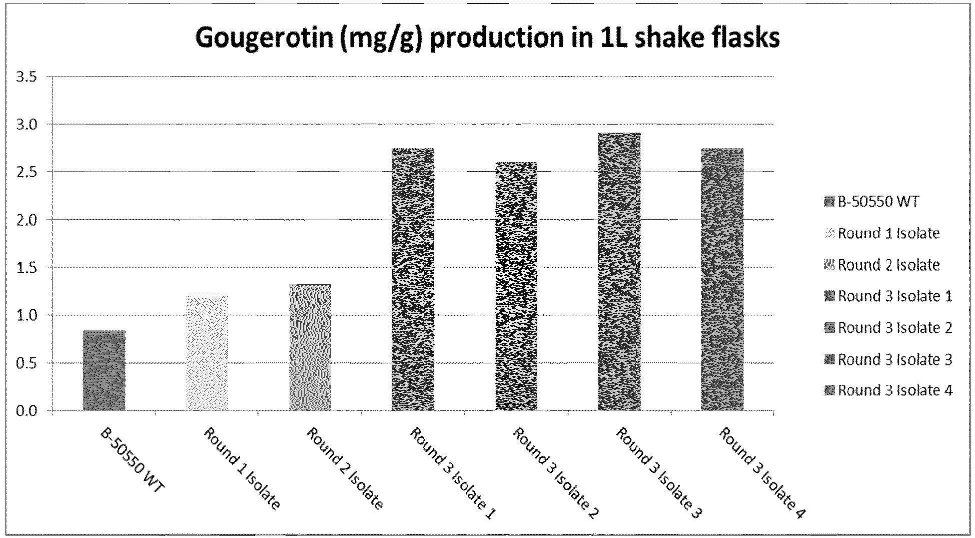

Selected mutants with higher gougerotin production and ability to sporulate on SFM agar plates were grown in 1-L baffled shake flasks and subsequently scaled up to 5 L Sartorius B-plus bioreactors and/or 20-L bioreactors containing Medium 2. See following figure.

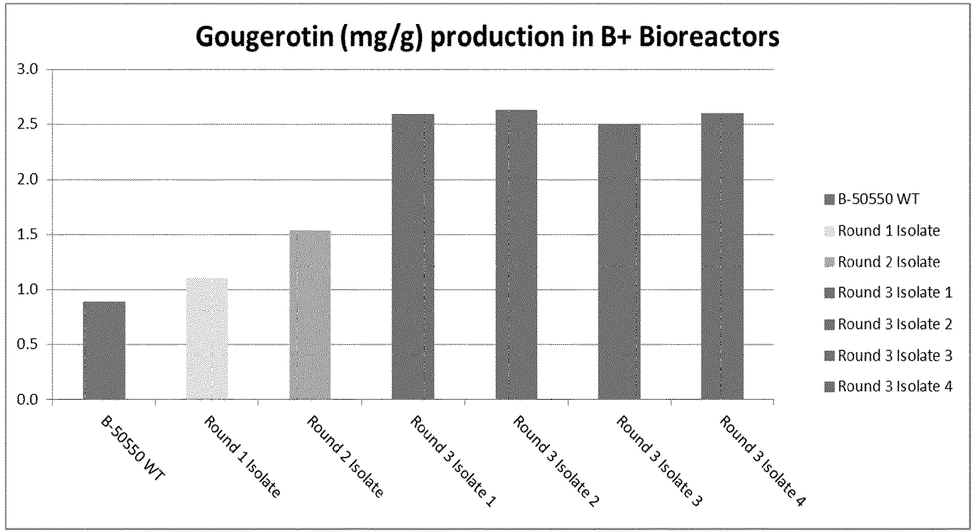

Bioreactor material for each isolate was also screened using the two spotted spider mite (TSSM) lab assay described above. It was confirmed that increases in gougerotin concentration correlate with TSSM bioactivity increases.

Unless defined otherwise, all technical and scientific terms herein have the same meaning as commonly understood by one of ordinary skill in the art to which this invention belongs. Although any methods and materials, similar or equivalent to those described herein, can be used in the practice or testing of the present invention, the preferred methods and materials are described herein. All publications, patents, and patent publications cited are incorporated by reference herein in their entirety for all purposes.

The publications discussed herein are provided solely for their disclosure prior to the filing date of the present application. Nothing herein is to be construed as an admission that the present invention is not entitled to antedate such publication by virtue of prior invention.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth and as follows in the scope of the appended claims.

## Claims

1. A fermentation broth obtained by cultivating a gougerotin producing *Streptomyces* strain, wherein the fermentation broth contains at least about 1 g/L gougerotin.

2. The fermentation broth of claim 1 wherein the fermentation broth contains at least about 2 g/L, of least about 3 g/L, of about at least 4 g/L, of about at least 5g/L, of about at least 6 g/L, of about at least 7 g/L or of about 8 g/L gougerotin.

3. The fermentation broth of claim 1 or claim 2, wherein the fermentation broth contains gougerotin in a concentration ranging from about 2 g/L to about 15 g/L.

4. The fermentation broth of any of the foregoing claims, wherein the *Streptomcyes* strain is a *Streptomyces microflavus* strain, a *Streptomyces graminearus* strainor a *Streptomcyes puniceus* strain, wherein the *Streptomyces microflavus* strain is preferably selected from the group consisting of *Streptomyces microflavus* strain NRRL B-50550 or a phytophagous-miticidal mutant strain derived therefrom and wherein the *Streptomyces graminearus* strain is preferably *Streptomyces graminearus* CGMCC 4.506.

5. A method of producing a fermentation broth of a gougerotin producing *Streptomyces* strain, wherein the fermentation broth contains at least about 1 g/L gougerotin, the method comprising cultivating the *Streptomyces* strain in a culture medium containing a digestible carbon source and a digestible nitrogen source under aerobic conditions, wherein the culture medium contains an amino acid at a concentration effective to achieve a gougerotin concentration of at least 1 g/L.

6. The method of claim 5, wherein the *Streptomyces* strain is cultivated in the culture medium until the culture medium contains gougerotin in a concentration of at least about 2 g/L, of at least about 3 g/L, of at least about 4 g/L, of at least about 5g/L, of at least about 6 g/L, of at least about 7 g/L or of at least about 8 g/L gougerotin.

7. The method of claim 5 or 6, wherein the *Streptomyces* strain is cultivated in the culture medium until the culture medium contains gougerotin in a concentration ranging from about 2 g/L to about 15 g/L gougerotin.

8. The method of any of claims 5 to 7, wherein the amino acid is selected from the group consisting of glycine, glutamic acid, glutamine and mixtures thereof.

9. The method of claim 8, wherein the culture medium contains the amino acid in an initial concentration of at least about 2 g/L.

10. The method of claim 8 or 9, wherein the culture medium contains glycine at an initial concentration of about 5g/L to about 15 g/L and/or wherein the culture medium contains glutamic acid in an initial concentration of about 5 g/L to about 15 g/L.

11. The method of any of claims 5 to 10, wherein the culture medium contains as carbon source a mixture of glucose and an oligosaccharide, wherein the oligosaccharide is preferably maltodextrin or dextrin.

12. The method of claim 11, wherein the initial maltodextrin concentration in the culture medium is about 50 g/L to about 100 g/L, or wherein the initial maltodextrin concentration in the culture medium is about 60 g/L to about 80 g/L.

13. The method of any of claims 11 to 12, wherein the initial glucose concentration in the culture medium is about 20 g/L to 60 g/L, or wherein the initial glucose concentration in the culture medium is about 30 g/L to about 50 g/L.

14. The method of any of the foregoing claims 5 to 13, wherein the *Streptomcyes* strain is a *Streptomyces microflavus* strain, wherein the *Streptomyces microflavus* strain is preferably selected from the group consisting of *Streptomyces microflavus* strain NRRL B-50550 or a phytophagous-miticidal mutant strain derived therefrom.

15. A composition comprising a fermentation product containing at least about 1 g/L gougerotin.
